(19) Europäisches Patentamt
European Patent Office
Office européen des brevets

(11) **EP 0 821 231 B1**

(12) **EUROPÄISCHE PATENTSCHRIFT**

(45) Veröffentlichungstag und Bekanntmachung des Hinweises auf die Patenterteilung:
**10.12.2003 Patentblatt 2003/50**

(51) Int Cl.[7]: **G01N 33/18**, G01N 35/10

(21) Anmeldenummer: **97112346.8**

(22) Anmeldetag: **18.07.1997**

(54) **Vorrichtung zur Analyse von Flüssigkeiten**

Apparatus for analysing liquids

Appareil pour l'analyse de liquides

(84) Benannte Vertragsstaaten:
**AT BE CH DE DK ES FI FR GB GR IE IT LI LU MC NL PT SE**

(30) Priorität: **24.07.1996 DE 19629835**

(43) Veröffentlichungstag der Anmeldung:
**28.01.1998 Patentblatt 1998/05**

(73) Patentinhaber: **ABB PATENT GmbH**
**68526 Ladenburg (DE)**

(72) Erfinder:
• **Binz, Dieter, Dr.**
  **69198 Schriesheim (DE)**
• **Vogel, Albrecht, Dr.**
  **76297 Stutensee (DE)**
• **Claesson, Brith**
  **72227 Västeras (SE)**
• **Brändle, Hubert**
  **8102 Oberengstringen (CH)**
• **Keeping, Sean**
  **West Croydon, Surrey CRO 2PY (GB)**

(74) Vertreter: **Miller, Toivo et al**
**ABB Patent GmbH**
**Postfach 1140**
**68520 Ladenburg (DE)**

(56) Entgegenhaltungen:
**WO-A-94/25875          DE-A- 2 404 871**
**DE-A- 3 842 734**

• **RINKE G ET AL: "PROZESSMESSGERAET FUER AMMONIUM, NITRAT UND PHOSPHAT IM ABWASSER" TECHNISCHES MESSEN TM,DE,R.OLDENBOURG VERLAG. MUNCHEN, Bd. 62, Nr. 12, 1. Dezember 1995 (1995-12-01), Seiten 462-465, XP000550483 ISSN: 0171-8096**
• **TAN T C: "MICROBIAL MEMBRANE-MODIFIED DISSOLVED OXYGEN PROBE FOR RAPID BIOCHEMICAL OXYGEN DEMAND MEASUREMENT" SENSORS AND ACTUATORS B,CH,ELSEVIER SEQUOIA S.A., LAUSANNE, Bd. B08, Nr. 2, 1. Mai 1992 (1992-05-01), Seiten 167-172, XP000286952 ISSN: 0925-4005**
• **PATENT ABSTRACTS OF JAPAN vol. 017, no. 269 (P-1544), 25. Mai 1993 (1993-05-25) & JP 05 010942 A (MITSUI ENG & SHIPBUILD CO LTD), 19. Januar 1993 (1993-01-19)**

**Beschreibung**

Die Erfindung bezieht auf eine Vorrichtung zur Analyse von Flüssigkeiten gemäß dem Oberbegriff des Patentanspruchs 1.

Solche Vorrichtungen kommen vermehrt bei der Untersuchung von Abwässern in Kläranlagen zum Einsatz.

Aus der Druckschrift "Prozessmessgerät für Ammonium, Nitrat und Phosphat im Abwasser", Rinke G. et al, TM Technische Mitteilungen, 62 (1995), Dezember, No 12, ist eine Vorrichtung bekannt, die ausschließlich für die Bestimmung des chemischen Sauerstoffbedarfs von Flüssigkeiten in Kläranlagen vorgesehen ist. Sie umfaßt ein gehäuse, innerhalb dessen eine Reaktionskammer angeordnet ist, der die zu untersuchende Flüssigkeit zuführbar ist, und der eine Messvorrichtung zugeordnet ist. Mittel zur Bestimmung des biologischen Sauerstoffbedarfs von Abwassern sind nicht vorgesehen. Die bekannte Vorrichtung muss neben dem Becken einer Kläranlage aufgestellt werden, deren Abwässer zu untersuchen sind. Proben des Abwassers werden einer Mischkammer in der Vorrichtung zugeführt, und dort mit Reagenzien vermischt. Die Menge an Ammonium, Nitrat und Phosphat, die in einem Abwasser enthalten ist, wird photometrisch ermittelt. Die Messungen werden mit drei Messsystemen für Nitrat, Ammonium bzw. Phosphat durchgeführt .

In der DE-A-24 04 871 ist ein System zur Gewässerüberwachung beschrieben, das mit einer Ausrüsterboje und einer Sensorboje versehen ist. Die Ausrüsterboje weist zwei Schwimmkörper auf, die über eine Plattform miteinander verbunden sind. Die Sensorboje kann von der Ausrüsterboje aus in das zu überwachende Gewässer abgesenkt werden. Mit Hilfe einer Kette, die an der Sensorboje befestigt ist, kann die Sensorboje in einer vorgebbaren Tiefe des Gewässers gehalten werden. Die Sensorboje ist mit Mess-Sensoren für die Überprüfung des Wasser ausgerüstet. Mit den Mess-Sensoren können die Temperatur, die Trübung, der pH-Wert und die Menge an gelösten Sauerstoff ermittelt werden. Die Sensoren werden hierfür mit dem zu untersuchenden Wasser beaufschlagt.

Aus der Druckschrift "Microbial membrane- modified dissolved oxygen probe for rapid biochemical oxygen demand measurement", Tan, T.C. et al, Sensors and Actuators B, 1992, No 2, pages 1667 to 172" ist eine Vorrichtung zur Bestimmung des biologischen Sauerstoffbedarfs bekannt.

In der WO-A-94 25875 ist eine Vorrichtung zur Analyse von Flüssigkeiten beschrieben. Zu der Vorrichtung gehört eine Probezelle, in der eine Membran angeordnet ist. In diese Zelle kann das zu untersuchende Abwasser fließen. Orthophosphat - Ionen und Orthophosphat-Moleküle, die in dem Abwasser enthalten sind, können über die Membran in eine Leitung gelangen, durch die eine Trägerflüssigkeit geleitet wird. Hierdurch wird die Trägerflüssigkeit in eine Probenflüssigkeit umgesetzt. Die aus der Probenzelle kommende Probenflüssigkeit wird einem Mischpunkt zugeführt, wo weitere Flüssigkeiten zugemischt werden können.

Aus der Druckschrift "Präzisions-Labor-Sauerstoffmessgeräte", Wissenschaftlich - Technische Werkstätten GmbH, sind Vorrichtungen zur Ermittlung des biologischen Sauerstoffbedarfs bekannt, die alle die Messung des Sauerstoffverbrauchs in Wasserproben zum Inhalt haben. Sie lassen sich unterteilen in Laborverfahren und On-line - Messverfahren. Die Laborverfahren sind genormt und in der DIN 38409 Teil 51 beschrieben. Es handelt sich um eine Verdünnungsmethode, mit der innerhalb von fünf Tagen der Gehalt an biologisch abbaubaren Substanzen in der Wasserprobe im Labor ermittelt wird.

In der Druckschrift "Biotechnologie", Berghof Labor- und Automationstechnik GmbH, 10/95/2000 ist ein Fermentationskalorimeter beschrieben, der die Wärmeproduktion metabolischer Prozesse misst. Hierfür wird das Wärmeprodukt des Fermentguts durch eine entsprechende Kühlung kompensiert. Die ermittelte Kühlrate ist proportional zum metabolischen Wärmeprodukt und damit auch proportional zur Stoffwechselaktivität der Biomasse. Der apparative Aufwand ist allerdings hoch, und entsprechende Geräte sind für den Einsatz als Prozessmessgerät zu teuer.

Aus der Druckschrift "Food Biosensor Analysis", Enzyme Thermistors for Food Analaysis, Bengt Danielsson, M. Dekker, New York, 1994 sind Enzymthermistoren bekannt, welche die Wärme messen, die entsteht, wenn eine immobilisierte Schicht aus Enzymen mit einem organischen Stoff reagiert. Die Wärme wird beispielsweise mittels zweier Absoluttemperatursensoren gemessen, die sich im Zu- oder Ablauf des Reaktionsgefäßes mit den immobilisierten Enzymen befinden.

In der Druckschrift "Development of an Integrated Thermal Biosensor for the Simultaneous Determination of Multiple Analytes", Analyst, January 1995, Vol. 120, ist ein Fließsystem beschrieben, bei dem eine Enzymsäule durchströmt wird, an deren Ende die Temperatur ermittelt wird. Als Referenz dient hierbei ein zweites System mit einer unbeschichteten Säule. Auch hierbei ist die Signaldifferenz der Signale von den beiden Temperatursensoren ein Maß für die Reaktionswärme.

Der Erfindung liegt die Aufgabe zugrunde, eine Vorrichtung zur Analyse von Flüssigkeiten aufzuzeigen, die mit der zu untersuchenden Flüssigkeit unmittelbar in Kontakt gebracht werden kann, wenig Platz beansprucht und sich leicht transportieren läßt.

Diese Aufgabe wird durch die Merkmale des Patentanspruchs 1 gelöst.

Die erfindungsgemäße Vorrichtung ist so konstruiert, dass damit das Abwasser in Kläranlagen (hier nicht dargestellt) auf seinen biologischen Sauerstoffbedarf und auf schadstoffhaltige Bestandteile sowie auf seinen Gehalt an Nährstoffen auf einfache Weise untersucht werden kann. Da die Vorrichtung aus Mikrobauteilen aufgebaut ist, weist sie sehr kleine Abmessungen auf, auf Grund derer sie auch leicht zu handhaben ist. Die Vorrichtung ist schwimmfähig und kann beispielsweise unmittelbar in den Abwasserbecken von Kläranlagen positioniert werden. Spezielle Zuleitungen für das zu untersuchende Abwasser sind nicht erforderlich. Die Vorrichtung ist mit mehreren Reaktionskammern versehen, so dass mit ihr der biologische Sauerstoffbedarf des Abwassers ermittelt und zudem auch festgestellt werden kann, ob sich Ammonium, Nitrat und Phosphat darin befinden. Durch die Verwendung einer größeren Anzahl von Reaktionskammern, Enzymen und/ oder Reagenzien kann die Analyse auf einfache Weise auf weitere Bestandteile ausgedehnt werden.

Weitere erfinderische Merkmale sind in den abhängigen Ansprüchen gekennzeichnet.

Die Erfindung wird nachfolgend an Hand von schematischen Zeichnungen näher erläutert.

Es zeigen:

Fig. 1    eine Vorrichtung zur Analyse von Abwasser,
Fig. 2    eine Variante der in Fig. 1 dargestellten Vorrichtung,
Fig. 3    eine vereinfachte Ausführungsform der Vorrichtung nach Fig. 1,
Fig. 4    eine Variante der in Fig. 3 gezeigten Ausführungsform.

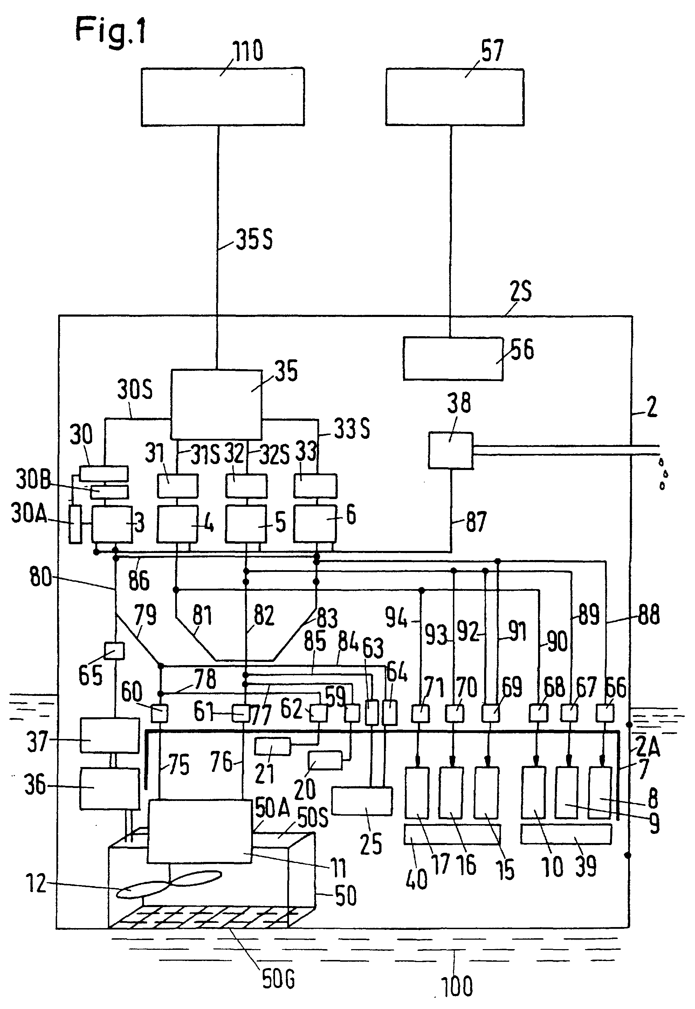

Die in Fig. 1 gezeigte Vorrichtung 1 weist ein schwimmfähiges Gehäuse 2, vier Reaktionskammern 3, 4, 5 und 6, eine Kassette 7, die drei Kammern 8, 9 10 für Enzyme, einen Filter 11, drei Kammern 15, 16, und 17 für Reagenzien, zwei Kammern für Kalibrierlösungen 20 und 21 sowie eine Kammer 25 für Pufferlösung umfaßt, einen Rotor 12, vier Meßvorrichtungen 30, 31, 32 und 33, einen Mikroprozessor 35, eine Wasseraufbereitungsvorrichtung 36, einen Frischwasserbehälter 37, eine Filtervorrichtung 38, eine Heizvorrichtung 39, eine Kühlvorrichtung 40 sowie einen Spannungsverteiler 56 auf. Das Gehäuses 2 ist schwimmfähig ausgebildet und kann direkt in das Abwasserbecken einer Kläranlage (hier nicht dargestellt) eingetaucht werden. Das zu untersuchende Abwasser 100 wird über das Filter 11 und zwei nachgeschaltete Pumpen 60 und 61 sowie die Zuleitungen 75, 79 und 80 bzw. die Zuleitungen 76 und 81, 76 und 82, 76 und 83 den Reaktionskammern 3 ,4, 5 und 6 zugeführt. Zu diesem Zweck ist das Filters 11 direkt in das Abwasser 100 getaucht. An Stelle des Filters 11 kann auch eine Membran (hier nicht dargestellt) zum Ansaugen des Abwassers 100 verwendet werden. Das Gehäuses 2 ist zum Ansaugen des Abwassers 100 an seinem unteren Ende, welches in das Abwasser 100 eintaucht, mit einem Behälter 50 versehen, der die Form eines Zylinders hat, oder der wie hier dargestellt die Form eines Quaders besitzt. Dieses Behälter 50 ist zur Aufnahme von Abwasser 100 vorgesehen. Er ist an seiner Oberseite und an seinen seitlichen Begrenzungsflächen geschlossen. An seiner Oberseite weist er eine Öffnung 50A auf, durch die das untere Ende des Filter 11 so gesteckt ist, daß dieses einige Millimeter in den Behälter 50 hineinragt. Die Unterseite des Behälters 50 ist mit einem Metallgitter 50G abgedeckt. Durch dieses Metallgitter 50G kann das Abwasser 100 in den Behälter 50 strömen. Die Maschen des Metallgitters 50G weisen eine solche Größe auf, daß höchstens feine Schwebteilchen mit dem Abwassers 100 in den Behälter 50 gelangen. Gleichzeitig werden mit dem Metallgitter 50G Fette (hier nicht dargestellt) von dem Filter 11 ferngehalten. Damit das Filter 11 nicht durch kleine Schwebteilchen (hier nicht dargestellt) zugesetzt wird, ist innerhalb des Behälters 50 der Rotor 12 installiert. Er ist wie Fig. 1 zeigt etwas versetzt zu dem Filter 11 angeordnet, so daß seine Rotorblätter das Abwasser 100 mit sehr hoher Geschwindigkeit an dem Filter 11 vorbeibewegen. Die Öffnung 50A ist so ausgebildet, daß nur das von dem Filter 11 und den nachgeschalteten Pumpen 60 und 61 angesaugte Abwasser 100 in die Vorrichtung 1 gelangen kann und das ungewünschte Einströmen größerer Mengen von Abwasser 100 verhindert wird.

Jede der hier dargestellten Reaktionskammern 3, 4, 5 und 6 hat ein Volumen, das höchstens 1cm$^3$ beträgt. Die Reaktionskammer 3 ist zur Bestimmung des biologischen Sauerstoffbedarfs des Abwassers 100 vorgesehen. In der Reaktionskammer 3 sind Bakterien (hier nicht dargestellt) enthalten. Diese sind in der Lage, organische Substanzen, die in dem Abwasser 100 enthalten sind, oxidativ umzusetzen. Bei diesen aeroben Prozessen wird Sauerstoff verbraucht und Biomasse und Wärme erzeugt. Damit die Bakterien beim Leeren der Reaktionskammer 3 nicht weggeschwemmt werden, sind sie auf Substraten (hier nicht dargestellt) immobilisiert. Bevorzugt werden Substrate verwendet, die als kleine Perlen geformt und beispielsweise aus Glas gefertigt sind. Die Bakterien sind in den Poren dieser Perlen angesiedelt. Vorzugsweise werden zwei oder mehrere Substrate verwendet, auf den unterschiedliche Bakterien angesiedelt sind. In der Reaktionskammer 3 sind beispielsweise Substrate mit Bakterien der Stämme Rhodococcus Erytropolis und Issatchenkia Orientalis immobilisiert. Die perlenförmigen Substrate mit einem Durchmesser von 0,1mm bis 1 mm werden mit Hilfe eines Netzes (hier nicht dargestellt) beim Entleeren der Reaktionskammer 3 zurückgehalten. Der Reaktionskammer 3 ist die Meßvorrichtung 30 zugeordnet. Diese weist zwei elektrochemisch arbeitende Sensoren 30 A und 30B auf, mit denen der Sauerstoffgehalt in dem Abwasser 100 gemessen werden kann. Der Sensor 30A ist so angeordnet, daß er den Sauerstoffgehalt des gerade in die Reaktionskammer 3 fließenden Abwassers 100 mißt. Nach dem Einfüllen einer definierten Menge von etwa weniger als 1cm$^3$ an Abwasser 100 wird die Reaktionskammer 3 mit Pufferlösung aus dem Behälter 25 aufgefüllt, die der Reaktionskammer 3 über die Zuleitungen 79, 80 und 84 mittels der Pumpe 64 zugeführt wird. Als Pufferlösung wird hierbei destilliertes Wasser oder ein Boratpuffer verwendet. Mit dieser Pufferlösung können auch die Reaktionskammern 4, 5 und 6 über die Zuleitungen 81 und 85 bzw. 82 und 85 bzw. 83 und 85 mit Hilfe der Pumpe 63 versorgt werden. Der zweite Sensor 30B ist so installiert, daß er den Sauerstoffgehalt des Abwassers 100 messen kann, nach dem die Bakterien die darin enthaltenen organischen Substanzen in Biomasse und Wärme umgesetzt und dabei Sauerstoff verbraucht haben. Aus der Menge des verbrauchten Sauerstoffs kann die Menge der organischen Substanzen im Abwasser 100 ermittelt werden. An Stelle der hier beschriebenen Meßvorrichtung 30 kann auch eine optisch arbeitende oder jeder andere hierfür geeignete Meßvorrichtung verwendet werden

Nach jeder Messung wird die Reaktionskammer 3 mit Reinwasser gespült. Das gilt auch für die drei anderen Reaktionskammern 4, 5 und 6. Dieses Wasser wird von der Wasseraufbereitungsvorrichtung 36 aus dem Abwasser 100 gewonnen und in dem Behälter 37 gespeichert. Der Aufbau der Wasseraufbereitungsvorrichtungen 36 gehört bereits zum Stand der Technik und wird deshalb hier nicht näher erläutert. Von dem Behälter 37 wird das Wasser über die Zuleitungen 80 und 86 mit Hilfe der Pumpe 65 den Reaktionskammern 3, 4, 5 und 6 zugeführt. Anstelle einer Spülung mit Reinwasser können die Reaktionskammern 3, 4, 5 und 6 auch mit Abwasser 100 gespült werden, das aus dem Filter 11 über die Leitungen 75, 76, 79, 80, 81, 82, 83 mittels der Pumpen 60 und 61 zugeführt wird. Die aus den Reaktionskammern 3, 4, 5 und 6 entleerten Flüssigkeiten werden über die Leitung 87 der Filtervorrichtung 38 und von dort der Kläranlage (hier nicht dargestellt) zugeführt.

Die Meßsignale der Meßvorrichtung 30 werden an den Mikroprozessor 35 weitergeleitet, mit dessen Signaleingang sie über die Signalleitung 30S verbunden ist. Dort werden die Meßsignale gespeichert und an einen Transmitter 110 weitergeleitet. Dieser ist außerhalb der Vorrichtung 1 in einer Warte (hier nicht dargestellt) installiert. Der Mikroprozessor 35 kann wie in Fig. 1 dargestellt über eine Signalleitung 35S mit dem Transmitter 110 verbunden werden. Es besteht jedoch auch die Möglichkeit, die Meßsignale mit einem Sender (hier nicht dargestellt) zu übertragen. Mit Hilfe des Transmitters 110 werden die Meßsignale ausgewertet und angezeigt. In dem Mikroprozessor 35 sind ferner Programme gespeichert, nach denen der Mikroprozessor 35 alle Pumpen der Vorrichtung 1 so steuert, daß die Reaktionskammer 3, 4, 5 und 6 zur richtigen Zeit immer mit den erforderlichen Flüssigkeiten versorgt werden.

Neben der Reaktionskammer 3 sind auch die Reaktionskammern 4, 5 und 6 für die Analyse des Abwassers 100 vorgesehen. Jeder dieser Reaktionskammern 4, 5 und 6 ist eine Meßvorrichtung 31, 32 und 33 zugeordnet, deren Meßsignale über je eine Signalleitung 31 S, 32S und 33S dem Mikroprozessor 35 zuführbar sind. Der Nachweis von Ammonium, Nitrat und Phosphat erfolgt in je einer der Reaktionskammern 4, 5 und 6 mit Hilfe von Enzymen und Reagenzien. Dabei wird die Reaktionskammer 4 zum Nachweis von Phosphat genutzt, während die Analyse des Abwasser 100 auf seinen Gehalt an Ammonium in der Reaktionskammer 5 erfolgt. In der Reaktionskammer 6 wird das Abwasser 100 auf seinen Nitratgehalt hin überprüft. Die Enzyme in Form von Nitratreductase, Glutamat Dehydrogenase und Pyruvatoxidase sind in jeweils einer der Kammern 8, 9 und 10 enthalten, während in die Kammer 15, 16 und 17 jeweils ein Reagenz in Form von NADH, alpha-Ketoglutarat und Pyruvat gefüllt ist. Wie Fig. 1 zeigt gehören die Kammern 8, 9, 10, 15, 16, 17 ,20, 21, 25 so wie das Filter 11 zu der Kassette 7. Diese ist so ausgebildet, daß sie über eine wasserdicht verschließbare Öffnung 2A im Gehäuses 2 jeder Zeit diesem entnommen und durch eine andere ersetzt werden kann. Ein solcher Austausch erfolgt vorzugsweise alle drei Monate, da die Inhalte der Kammern 8, 9, 10, 15, 16, 17 ,20, 21, 25 gerade so bemessen sind, daß sie für etwa 100 Tage bzw. 9660 Untersuchungen ausreichen, wenn davon ausgegangen wird, daß jede Stunde vier Messungen durchgeführt werden. Das Fassungsvermögen der Kammern 8, 9 und 10 für die Enzyme beträgt 26ml, 1,1ml und 0,2ml. Die Kammern 15, 16 und 17 für die Reagenzien weisen ein Fassungsvermögen von 4,2ml, 1,3ml und 0,1ml auf. Die Kammern 8, 9, 10, 15, 16, 17 ,20, 21, 25 der Kassette 7 sind mit Anschlußelementen (hier nicht dargestellt) für die Zuleitungen 75 bis 94 versehen, so daß eine einfaches Lösen oder Verbinden möglich ist. An Stelle dieser Kassette 7 können alle Zuleitungen auch über schlauchartige Verbindungsleitungen (hier nicht dargestellt) an Vorratsbehälter (hier nicht dargestellt) angeschlossen werden, die außerhalb des Gehäuses 2 auf einem festen Untergrund aufgestellt sind. Das Gehäuse 2 ist für diesen Fall mit wasserdicht verschließbaren Öffnungen (hier nicht dargestellt) für die Durchführung der schlauchartigen Verbindungsleitungen versehen.

Die Verbindung der Reaktionskammern 4, 5 und 6 mit den Kammern 8, 9, 10, 15, 16 und 17 erfolgt über Zu- und Verbindungsleitungen. Wie Fig. 1 zeigt ist die Reaktionskammer 6 über eine Zuleitung 88 an die Kammer 8 angeschlossen, während die Reaktionskammer 5 über die Zuleitung 89 mit der Kammer 9 verbunden ist. Die Reaktionskammer 4 steht über die Zuleitung 90 mit der Kammer 10 in Verbindung. In jede der Zuleitungen 88, 89 und 90 ist eine Pumpe 66, 67 und 68 eingebaut. An die Zuleitung 88 der Reaktionskammer 6 ist über eine Verbindungsleitung 91 die Kammer 15 angeschlossen. Die Kammer 15 steht über eine zweite Verbindungsleitung 92 mit der Reaktionskammer 5 in Verbindung. Über eine Verbindungsleitung 93 ist die Kammer 16 auch an die Reaktionskammer 5 anschlossen. Über eine Verbindungsleitung 94 ist die Kammer 17 mit der Reaktionskammer 4 verbunden. Jede der Kammern 15, 16 und 17 ist eine Pumpe 69, 70 und 71 nachgeschaltet. Ferner ist eine Kammer 20 mit Kalibrierlösung über die Zuleitungen 77 und 81 bzw. 77 und 82 bzw. 77 und 83 an die Reaktionskammern 4, 5 und 6 angeschlossen, während eine zweite Kammer 21, die ebenfalls Kalibrierlösung enthält, über die Zuleitungen 78, 79 und 80 an die Reaktionskammer 3 angeschlossen ist. Beiden Kammern 20 und 21 ist jeweils eine Pumpe 59 und 62 nachgeschaltet.

Für den Transport aller Flüssigkeiten, welche für die Analysen benötigt werden, können an Stelle der oben beschriebenen Zu- und Verbindungsleitungen 77 bis 94 und Pumpen 60 bis 71 auch Zuleitungen verwendet werden (hier nicht dargestellt), die im Inneren eine kapillare Struktur (hier nicht dargestellt) aufweisen. Die Steuerung der zu den Reaktionskammern 3, 4, 5 und 6 zu transportierenden Flüssigkeitsmengen kann mit Hilfe von Ventilen (hier nicht dargestellt) erfolgen, die in diese Zuleitungen eingebaut und durch den Mikroprozessor 35 angesteuert werden.

Die Kalibrierlösung 20 dient dazu, in regelmäßigen Abständen, vorzugsweise einmal täglich, eine Kalibrierung der Meßvorrichtungen 31, 32, 33 für Ammonium, Nitrat, Phosphat vorzunehmen. Sie besteht aus einer wässrigen Lösung, die genau bekannte Konzentrationen an Nitrat, Ammonium und Phosphat enthält, bevorzugt am oberen Ende des gewünschten Meßbereichs von 100mg/l. Zur Kalibrierung der Meßvorrichtungen 31, 32, 33 wird im Kalibrierzyklus die Kalibrierlösung anstelle des Abwassers in die Reaktionskammern 4, 5 und 6 geleitet. Die von den Meßvorrichtungen 31, 32, 33 ermittelten Werte für Nitrat, Ammonium und Phosphat werden mit den bekannten Konzentrationen in dem Mikroprozessor 35 verglichen und gespeichert. Nachfolgende Messungen werden dann entsprechend korrigiert.

Die Kalibrierlösung 21 dient dazu, in regelmäßigen Abständen, vorzugsweise einmal täglich, eine Kalibrierung der Meßvorrichtung 30 für den biologischen Sauerstoffbedarf vorzunehmen. Sie besteht aus einer gepufferten wässrigen Lösung mit einem genau definierten Gehalt an Glucose oder einer genau bekannten Mischung aus Glucose und Glutaminsäure, und weist einen genau definierten Anteil an biologischem Sauerstoffbedarf auf, der vorzugsweise zwischen 200 und 500 mg/l liegt. Zur Kalibrierung der Meßvorrichtung 30 wird die Kalibrierlösung im Kalibrierzyklus anstelle des Abwassers 100 in die Reaktionskammer 3 geleitet. Der von der Meßvorrichtung 30 ermittelte Wert für den biologischen Sauerstoffbedarf wird mit der bekannten Konzentration im Mikroprozessor 35 verglichen und gespeichert. Nachfolgende Messungen werden dann entsprechend korrigiert.

Damit die Enzyme und Reagenzien immer den Temperaturen ausgesetzt sind, die für ihre Erhaltung erforderlich sind, ist den Kammern 8, 9 und 10 die Heizvorrichtung 39 und den Kammern 15, 16 und 17 die Kühleinrichtung 40 zugeordnet.

Für den Nachweis von Nitrat bei einer angenommenen maximalen Konzentration von 100mg/l Nitrat im Abwasser 100 werden 10 µl Abwassers 100 in die Reaktionskammer 6 gepumpt. Anschließend werden aus der Kammer 8 0,1µl Nitratreductase, das dort in einer Konzentration von 10mg/l vorrätig gehalten wird, und aus der Kammer 15 5,3µl NADH, das dort in einer Konzentration von 1,6 mMol/l vorrätig gehalten wird, in die Reaktionskammer 6 eingeleitet.

Ist in dem Abwasser Nitrat enthalten, so läuft folgende Reaktion ab:

$$\text{Nitrat + NADH+ Nitratreductase} \rightarrow \text{Nitrite + NAD}^{\oplus} + H_2O$$

Mit Hilfe der Meßvorrichtung 33, die hierbei aus Dickschichtsensoren aufgebaut ist, kann $NAD^{\oplus}$ und damit die Existenz von Nitrat im Abwasser nachgewiesen werden.

Der Nachweis von Ammonium wird in der Reaktionskammer 5 durchgeführt. Zunächst werden wieder 10 µl Abwasser 100, 0,14µl alpha-Ketoglutarat in einer Konzentration von 50mg/l und 0,13µl Glutamat Dehydrogenase in einer Konzentration von 10mg/l in die Reaktionskammer 5 gepumpt. Ist Ammonium im Abwasser 100 enthalten, so läuft dort folgende Reaktion ab:

$$\text{Ammonium + NADH + alpha-Ketoglutarat + Glutamat Dehydrogenase} \rightarrow \text{L-Glutamat + NAD}^{\oplus}$$

Mit Hilfe der Meßvorrichtung 32, die auch aus Dickschichtsensoren aufgebaut ist, kann $NAD^{\oplus}$ und damit die Existenz von Nitrat im Abwasser nachgewiesen werden.

Der Nachweis von Phosphat erfolgt in der Reaktionskammer 4. Nach dem Einfüllen von 10 µl Abwasser 100, 0,001 µl Pyruvat in einer Konzentration von 1Mol/l und 0,02µl Pyruvatoxidase in einer Konzentration von 110mg/l läuft in der Reaktionskammer 4, unter der Voraussetzung in dem Abwasser 100 ist Phosphat enthalten, folgende Reaktion ab:

$$\text{Phosphat + Pyruvat + Pyruvatoxidase} \rightarrow \text{Acotylphosphat + CO}_2 + H_2O_2$$

Mit Hilfe der Meßvorrichtung 31, die in gleicher Weise ausgebildet ist wie die Meßvorrichtung 33, wird $H_2O_2$ und damit Phosphat im Abwasser nachgewiesen.

Die Meßsignale der Meßvorrichtungen 31, 32 und 33 werden an den Mikroprozessor 35 und von dort an den Transmitter 110 zur Auswertung weitergeleitet.

Die Existenz von Nitrat, Ammonium und Phosphat im Abwasser 100 kann auch ausschließlich mit Reagenzien nachgewiesen werden. Hierfür werden dann bevorzugt solche verwendet, die eine Einfärbung der in den Reaktionskammern 4, 5 und 6 enthaltenen Flüssigkeiten bewirken, wenn Nitrat, Ammonium bzw. Phosphat im Abwasser 100 enthalten sind. In diesem Fall werden optisch arbeitende Meßvorrichtungen 31, 32 und 33 verwendet. An Stelle der hier beschriebenen Meßvorrichtungen können auch alle anderen Vorrichtungen dieser Art verwendet werden, wenn sie den Anforderungen für diese Messungen genügen.

Nach jeder Messung werden die Reaktionskammern 4, 5 und 6 mit Reinwasser aus dem Behälter 37 oder mit Abwasser aus dem Filter 11 gespült. Die Zeitintervalle in denen die Analysen durchgeführt werden sind in dem Mikroprozessor gesteuert. Von ihm werden alle Pumpen 60 bis 71 gesteuert, die zu diesem Zweck über Signalleitungen (hier nicht dargestellt) mit dem Mikroprozessor 35 in Verbindung stehen. Für die elektrische Versorgung ist der Spannungsverteiler 56 vorgesehen, dem die elektrische Spannung von einer Spannungsquelle 57 zugeführt wird, die außerhalb des Gehäuses 2 angeordnet ist. An den Spannungsverteiler 57 sind der Rotor 12, der Mikroprozessor 35, die Heizvorrichtung 39 und die Kühlvorrichtung 40 sowie die Pumpen 59 bis 71 angeschlossen. An Stelle einer externen Spannungsquelle 56 können auch photoelektrochemische Zellen (hier nicht dargestellt) auf der Oberfläche 2S des Gehäuses 2 angeordnet werden, von denen dann die erforderliche Spannung geliefert werden kann.

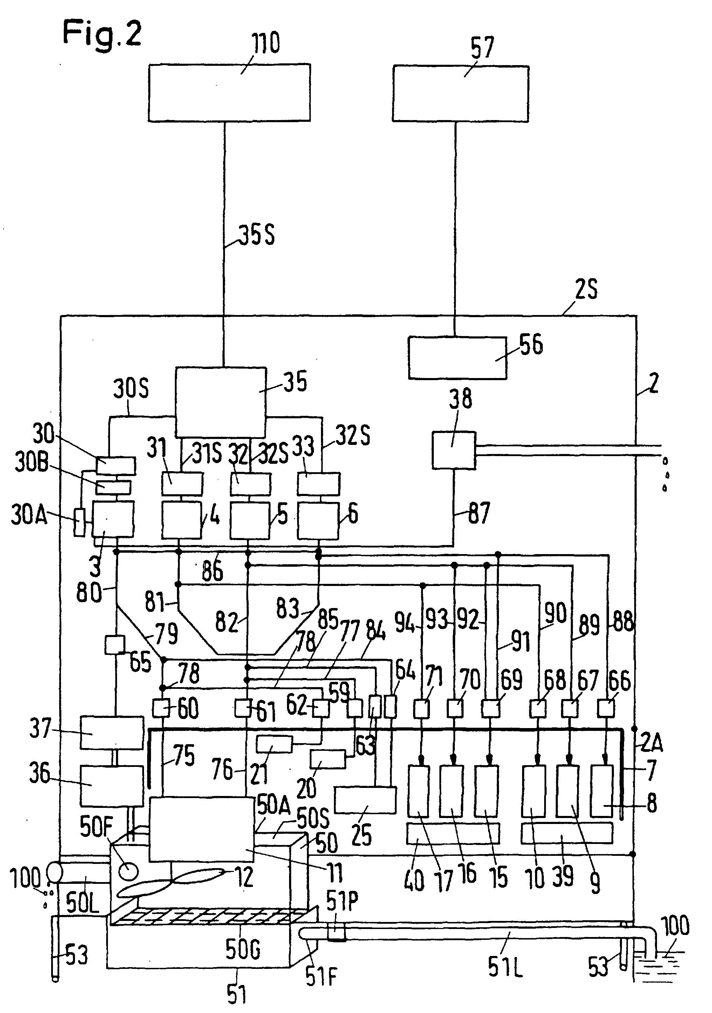

Fig. 2 zeigt eine Variante der in Fig. 1 dargestellten Vorrichtung 1, aber keine Ausführungsform der vorliegenden Erfindung. Beide Vorrichtungen sind nahezu baugleich. Gleiche Bauelemente sind deshalb mit den gleichen Bezugszeichen versehen. Der einzige Unterschied zwischen den beiden Vorrichtungen 1 besteht darin, daß die Vorrichtung 1 nach Fig. 2 außerhalb der zu untersuchenden Flüssigkeit betrieben und angeordnet werden kann. Zu diesem Zweck wird gegen die Unterseite des Behälters 50 ein wannenförmiges Bauelement 51 mit gleichem Querschnitt gesetzt und wasserdicht mit dem Behälter 50 verbunden. Das Bauelement 51 ist an einer Seite mit einer Öffnung 51 F versehen, an die eine Leitung 51 L angeschlossen ist. Der Behälter 50 weist im oberen Bereich der Seite, die der Öffnung 51 F gegenüber liegt, ebenfalls eine Öffnung 50F auf, an die auch eine Leitung 50L angeschlossen ist. In die Leitung 51 L ist eine Pumpe 51 P integriert. Über die Leitung 51 L wird Abwasser 100 aus dem Becken einer Kläranlage (hier nicht dargestellt) angesaugt. Die Förderleistung der Pumpe 51 P ist so bemessen, daß der Behälter 50 immer bis zu seiner Oberkante 50S mit Abwasser 100 gefüllt ist, so daß der Bereich des Filters 11, der in den Behälter 50 ragt, in das Abwasser 100 eingetaucht ist. Nicht benötigtes Abwasser 100 fließt über die Leitung 50L wieder aus dem Behälter 50 in die Kläranlage (hier nicht dargestellt) zurück. Zusätzliche Stützelemente 53, die an der Unterseite des Gehäuses 2 in seinem Randbereich befestigt sind, erhöhen die Standfestigkeit der Vorrichtung auf festem Untergrund (hier nicht dargestellt).

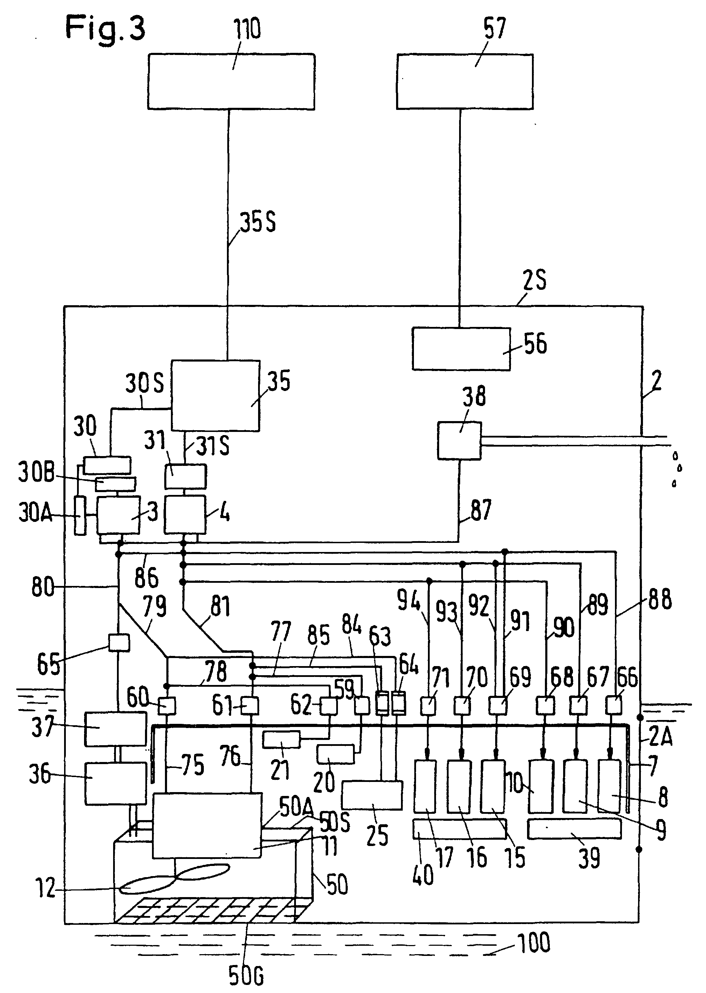

Fig. 3 zeigt ein vereinfachte Vorrichtung 1, die jedoch im Kern mit der Vorrichtung 1 gemäß Fig. 1 übereinstimmt. Gleiche Bauteile sind deshalb auch hierbei mit gleichen Bezugszeichen versehen. Der Unterschied besteht lediglich darin, daß neben der Reaktionskammer 3 zur Bestimmung des biologischen Sauerstoffbedarfs nur eine weitere Reaktionskammer 4 vorgesehen ist. In ihr wird das Abwasser 100 nacheinander auf Nitrat, Ammonium und Phosphat untersucht. Nach jeder Analyse wird die Reaktionskammer 4 zunächst mit Reinwasser oder mit Abwasser gespült. Bei der hier dargestellten Ausführungsform ist die Reaktionskammer 4 deshalb mit allen Vorratsbehältern 8, 9, 10 sowie 15,16,17 für die Enzyme und Reagenzien über die Zu- und Verbindungsleitungen 88 bis 94 verbunden. In alle Zuleitungen 88 bis 94 sind auch hier Pumpen 66 bis 71 eingebaut. Die richtig zeitlich abgestimmte Zuführung des Abwassers 100, der Enzyme, Reagenzien, Pufferlösungen, Kalibrierlösungen und des Reinwassers wird von dem Mikroprozessor 35 aus gesteuert, der die Pumpen nach einem Programm betätigt, das in ihm gespeichert ist.

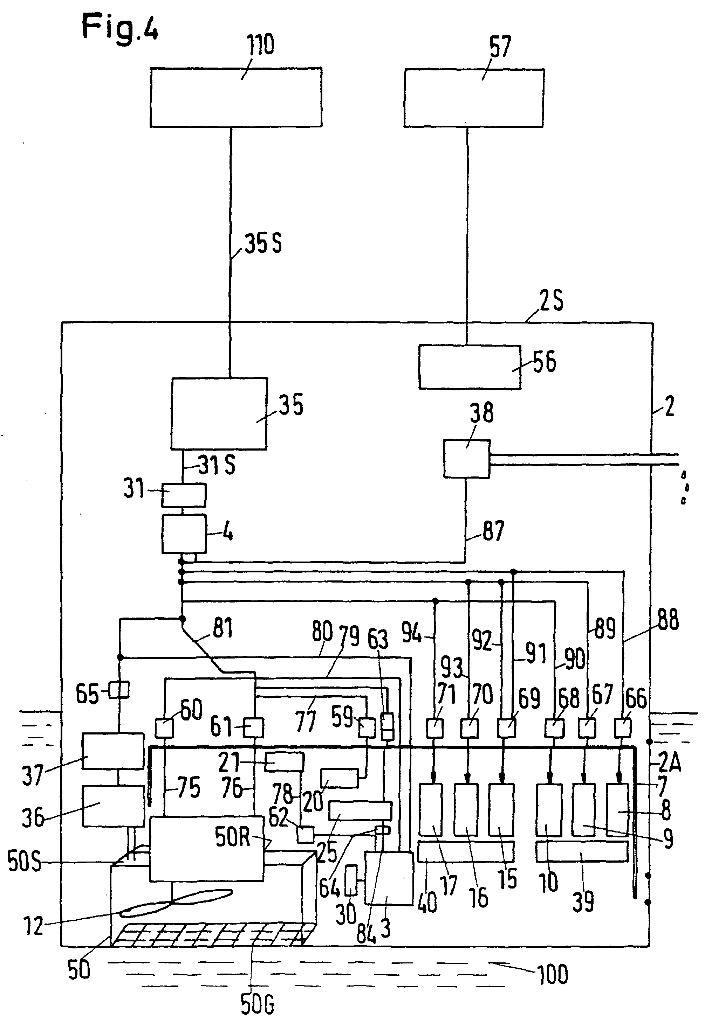

Die in Fig. 4 dargestellt Vorrichtung 1 unterschiedet sich von der Ausführungsform nach Fig. 3 lediglich dadurch, daß die Reaktionskammer 3 für die Bestimmung des biologischen Sauerstoffbedarfs in der Kassette 7 angeordnet ist. Das hat den Vorteil, daß mit jedem Wechsel der Kassette 7 auch die Reaktionskammer 3 ausgetauscht wird. Hiermit wird sichergestellt, daß nach etwa 100 Tagen die Bakterien (hier nicht dargestellt) in der Reaktionskammer 3 erneuert werden. Damit wird die Zuverlässigkeit der Messungen verbessert. Der Reaktionskammer 3 wird auch hierbei wie bei allen anderen Ausführungsformen das Abwasser 100 über die Zuleitungen 75 und 79 mit Hilfe der Pumpe 60 zugeführt. Der Anschluß an den Behälter 37 mit Reinwasser erfolgt über die Zuleitung 80. Die Pufferlösung aus der Kammer 25 wird über die Zuleitung 84 und die Pumpe 64 und die Kalibrierlösung aus der Kammer 21 wird ebenso wie bei allen anderen Ausführungsformen über die Zuleitung 78 und die Pumpe 62 zugeführt. Die Meßvorrichtung 30, die der Reaktionskammer 3 zugeordnet ist, steht auch hier mit dem Mikroprozessor 35 über eine Signalleitung (hier nicht dargestellt) in Verbindung. Die hier beschriebene Anordnung der Reaktionskammer 3 in der Kassette 7 ist auch bei allen oben beschriebenen Ausführungsformen möglich.

## Patentansprüche

1.  Vorrichtung zur Untersuchung von Flüssigkeiten, umfassend ein nach außen begrenzendes Gehäuse (2), innerhalb dessen Reaktionskammern (3, 4, 5, 6) angeordnet sind denen die zu untersuchende Flüssigkeit (100) zuführbar ist, und denen jeweils eine Messvorrichtung (30, 31, 32, 33) zugeordnet ist, wobei mindestens eine Reaktionskammer (3) für die Bestimmung des biologischen Sauerstoffbedarfs und wenigstens eine weitere Reaktionskammer (4, 5, 6) zur Bestimmung von weiteren Bestandteilen der Flüssigkeit (100) vorgesehen sind, wobei das Gehäuse (2) schwimmfähig ausgebildet und direkt innerhalb der zu untersuchenden Flüssigkeit (100) positionierbar ist, und das Gehäuse (2) einen einseitig offenen Behälter 50 aufweist, der bei gebrauch der Vorrichtung in die zu untersuchende Flüssigkeit (100) hinein ragt und in den ein Filter (11)oder eine Membran so gesteckt ist, dass von dort aus die zu untersuchende Flüssigkeit (100) in jede Reaktionskammer (3, 4, 5 und 6) leitbar ist, und dass jeder Reaktionskammer (3, 4, 5, 6) Flüssigkeiten für die Analyse aus Vorratsbehältern (8, 9, 10, 15, 16, 17, 20, 21, 25) zuführbar sind, die in dem Gehäuse (2) oder außerhalb angeordnet sind.

2. Vorrichtung nach Anspruch 1, worin die zu untersuchende Flüssigkeit (100) von dem Filter (11) oder der Membran aus mittels nachgeschalteter Pumpen (60 und 61) und Zuleitungen (79 und 80, 75 und 81, 76 und 82, 76 und 83) einer jeden Reaktionskammer (3,4, 5, 6) zuführbar ist.

3. Vorrichtung nach Ansprüch 1, worin die Vorratsbehälter (8, 9, 10, 15, 16, 17, 20, 21, 25) als Kammern ausgebildet und in einer Kassette (7) integriert sind, die lösbar in das Gehäuse (2) eingesetzt ist.

4. Vorrichtung nach Anspruch 3, worin die Reaktionskammer (3) für die Bestimmung des biologischen Sauerstoffbedarf innerhalb oder ausserhalb der Kassette (7) in dem Gehäuse (2) angeordnet ist, und wobei neben der Reaktionskammer (3) für die Bestimmung des biologischen Sauerstoffbedarfs drei weitere Reaktionskammern (4, 5 und 6) vorgesehen sind.

5. Vorrichtung nach einem der Ansprüche 3 oder 4, worin die Kassette (7) mit Kammern (8, 9, 10, 15, 16, 17, 20, 21, 25) für Enzyme und/oder Reagenzien, Kalibrierlösungen und Pufferlösungen sowie mindestens einem Filter (11 ) und/oder einer Membran versehen ist, der/die teilweise in dem Behälter (50) angeordnet ist, um dort in die zu untersuchende Flüssigkeit (100) getaucht zu werden, die über ein metallisches Gitter (50G) in den Behälter (50) leitbar und mit einem im Behälter (50) installierten Rotor (12) in Bewegung gehalten ist.

6. Vorrichtung nach einem der Ansprüche 1 bis 5, worin Enzyme und/oder Reagenzien, Kalibrierlösungen, Pufferlösungen und Reinwasser den Reaktionskammern (3, 4, 5 und 6) über Zu- und Verbindungsleitungen (77, 78, 79, 84, 85, 88, 89, 90, 91, 92, 93 und 94) sowie Pumpen (59, 62, 63, 64, 65, 66, 67, 68, 69, 70, 71 ) zuführbar sind.

7. Vorrichtung nach Anspruch 1, wobei die Vorratsbehälter (8, 9, 10, 15, 16, 17, 20, 21, 25) außerhalb des gehäuses (2) aufgestellt sind und über schlauchartige Zuführungen mit Zu- und Verbindungsleitungen (77, 78, 79, 84, 85, 88, 89, 90, 91, 92, 93 und 94 ) in dem Gehäuse (2) verbunden sind.

8. Vorrichtung nach Anspruch 1, wobei die Größe der Vorratsbehälter (8, 9, 10, 11, 15, 16, 17), die innerhalb des Gehäuses (2) angeordnet sind, so bemessen ist, dass ihr Fassungsvermögen an Enzymen und/oder Reagenzien, Pufferlösungen und Kalibrierlösungen für mindestens 9660 Untersuchungen ausgelegt ist, und dass die Größe der Reaktionskammern (3, 4, 5 und 6) höchstens 1 cm$^3$ beträgt.

9. Vorrichtung nach Anspruch 1, wobei in dem Gehäuse (2) ein Vorratsbehälter (37) für Reinwasser und eine damit verbundene Wasseraufbereitungsvorrichtung (36) installiert sind, der Abwasser (100) zuführbar ist.

10. Vorrichtung nach einem der Ansprüche 1 bis 6, wobei aus den Reaktionskammern (3, 4, 5 und 6) abgeleitete Flüssigkeiten einer Filtervorrichtung (38) zuführbar von dort aus dem Gehäuse (2) nach außen ableitbar sind.

11. Vorrichtung nach Anspruch 6, umfassend eine Spannungsquelle (57), die außerhalb des Gehäuses (2) installiert ist, und einen in dem gehäuse (2) angeordneten Spannungsverteiler (56), der mit der Spannungsquelle (57) verbunden ist, wobei mindestens ein Mikroprozessor (35) für die Steuerung der Pumpen (59, 61, 62, 63, 64, 65, 66, 67, 68, 69, 70, 71) und für die Speicherung und Weiterleitung von Messsignalen der Messvorrichtungen (31, 32 und 33) an einen Transmitter (110) vorgesehen ist.

## Claims

1. Device for the investigation of liquids, comprising an externally delimiting housing (2), inside which reaction chambers (3, 4, 5, 6) are arranged, to which can be fed the liquid (100) to be investigated, and which are each assigned a measuring device (30, 31, 32, 33), at least one reaction chamber (3) being intended for the determination of the biological oxygen demand and at least one further reaction chamber (4, 5, 6) being intended for the determination of further constituents of the liquid (100), the housing (2) being designed to be able to float and to be able to be positioned directly inside the liquid (100) which is to be investigated, and the housing (2) having a container (50) which is open on one side and which, when the device is in use, projects into the liquid (100) which is to be investigated and into which a filter (11) or a membrane is fitted in such a way that, from there, the liquid (100) to be investigated can be passed into each reaction chamber (3, 4, 5 and 6), and that liquids for analysis can be fed to each reaction chamber (3, 4, 5, 6) from storage containers (8, 9, 10, 15, 16, 17, 20, 21, 25) which are disposed in the housing (2) or outside.

2. Device according to Claim 1, wherein the liquid (100) to be investigated can be fed to each reaction chamber (3, 4, 5, 6) from the filter (11) or the membrane by means of downstream pumps (60 and 61) and supply lines (79 and 80, 75 and 81, 76 and 82, 76 and 83).

3. Device according to Claim 1, wherein the storage containers (8, 9, 10, 15, 16, 17, 20, 21, 25) are designed as chambers and are integrated in a cassette (7) which is removably inserted into the housing (2).

4. Device according to Claim 3, wherein the reaction chamber (3) for determining the biological oxygen demand is arranged inside or outside the cassette (7) in the housing (2), and three further reaction chambers (4, 5 and 6) being provided in addition to the reaction chamber (3) for determining the biological oxygen demand.

5. Device according to one of Claims 3 or 4, wherein the cassette (7) is provided with chambers (8, 9, 10, 15, 16, 17, 20, 21, 25) for enzymes and/or reagents, calibration solutions and buffer solutions as well as at least one filter (11) and/or a membrane, part of which is disposed in the container (50) in order to be immersed there in the liquid (100) to be investigated, which can be passed via a metallic grid (50G) into the container (50) and is maintained in motion by a rotor (12) installed in the container (50).

6. Device according to one of Claims 1 to 5, wherein enzymes and/or reagents, calibration solutions, buffer solutions and pure water can be fed to the reaction chambers (3, 4, 5 and 6) via supply lines and connecting lines (77, 78, 79, 84, 85, 88, 89, 90, 91, 92, 93 and 94) and pumps (59, 62, 63, 64, 65, 66, 67, 68, 69, 70, 71).

7. Device according to Claim 1, in which the storage containers (8, 9, 10, 15, 16, 17, 20, 21, 25) are set up outside the housing (2) and are connected via hose-type lines to supply and connecting lines (77, 78, 79, 84, 85, 88, 89, 90, 91, 92, 93 and 94) in the housing (2).

8. Device according to Claim 1, in which the size of the storage containers (8, 9, 10, 11, 15, 16, 17) which are disposed within the housing (2) is dimensioned in such a way that their capacity for enzymes and/or reagents, buffer solutions and calibration solutions is designed for at least 9660 investigations, and in that the size of the reaction chambers (3, 4, 5 and 6) is at most 1 cm$^3$.

9. Device according to Claim 1, in which a storage container (37) for pure water and a water processing device (36) connected to it, to which waste water (100) can be fed, are installed in the housing (2).

10. Device according to one of Claims 1 to 6, in which liquids derived from the reaction chambers (3, 4, 5 and 6) can be fed to a filter device (38) and can from there be branched off out of the housing (2) to the outside.

11. Device according to Claim 6, comprising a voltage source (57) which is installed outside the housing (2), and a voltage distributor (56) which is arranged in the housing (2) and is connected to the voltage source (57), at least one microprocessor (35) for the control of the pumps (59, 61, 62, 63, 64, 65, 66, 67, 68, 69, 70, 71) and for the storage and transmission of measurement signals of the measuring devices (31, 32 and 33) to a transmitter (110) being provided.

**Revendications**

1. Dispositif pour l'analyse de liquides comprenant un boîtier (2) formant une délimitation vers l'extérieur, à l'intérieur duquel sont disposées des chambres de réaction (3, 4, 5, 6) dans lesquelles un liquide à analyser (100) peut laissant peut être amené et à chacune desquelles est associé un dispositif de mesure (30, 31, 32, 33), une chambre de réaction (3) au moins étant prévue pour la détermination de la demande biologique en oxygène et au moins une autre chambre de réaction (4, 5, 6) étant prévue pour la détermination d'autres composants du liquide (100), le boîtier (2) étant conçu pour pouvoir flotter et pouvant être positionné directement à l'intérieur du liquide à analyser (100), et le boîtier (2) présentant un récipient (50) ouvert d'un côté, qui dépasse à l'intérieur du liquide à analyser (100) pendant l'utilisation de dispositif et dans lequel est introduit un filtre (11) ou une membrane de telle sorte que le liquide à analyser (100) peut être amené de celui-ci à chaque chambre de réaction (3, 4, 5 et 6), et en ce que des liquides peuvent être amenés pour analyse dans chaque chambre de réaction (3, 4, 5, 6) à partir de réservoirs (8, 9, 10, 15, 16, 17, 21, 25) qui sont disposés dans le boîtier (2) ou à l'extérieur.

2. Dispositif selon la revendication 1, **caractérisé en ce que** le liquide à analyser (100) peut être amené du filtre (11) ou de la membrane à chaque chambre réaction (3, 4, 5, 6) au moyen de pompes (60 et 61) montées en aval et de conduites (79 et 80, 75 et 81, 76 et 82, 76 et 83).

3. Dispositif selon la revendication 1, **caractérisé en ce que** les réservoirs (8, 9, 10, 15, 16, 17, 21, 25) sont conçus comme des chambres et intégrés dans une cassette (7) qui est introduite de façon amovible dans le boîtier (2).

4. Dispositif selon la revendication 3, **caractérisé en ce que** la chambre de réaction (3) pour la détermination de la demande biologique en oxygène est disposée à l'intérieur ou à l'extérieur de la cassette (7) dans le boîtier (2) et trois autres chambres de réaction (4, 5 et 6) sont prévues en plus de la chambre de réaction (3) pour la détermination de la demande biologique en oxygène.

5. Dispositif selon l'une quelconque des revendications 3 ou 4, **caractérisé en ce que** la cassette (7) est pourvue de chambres (8, 9, 10, 15, 16, 17, 20, 21, 25) pour des enzymes et/ou des réactifs, des solutions de calibration et des solutions tampons ainsi que d'au moins un filtre (11) et/ou une membrane, qui est partiellement disposé dans le récipient (50) pour être plongé dans celui-ci dans le liquide à analyser (100), lequel peut être amené dans le récipient (50) en passant par une grille métallique (50G) et maintenu en agitation par un rotor (12) installé dans le récipient (50).

6. Dispositif selon l'une quelconque des revendications 1 à 5, **caractérisé en ce que** des enzymes et/ou des réactifs, des solutions de calibration, des solutions tampons et de l'eau pure peuvent être amenés aux chambres de réaction (3, 4, 5 et 6) par des conduites d'arrivée et de communication (77, 78, 79, 84, 85, 88, 89, 90, 91, 92, 93 et 94) ainsi que des pompes (59, 62, 63, 64, 65, 66, 67, 68, 69, 70, 71).

7. Dispositif selon la revendication 1, **caractérisé en ce que** les réservoirs (8, 9, 10, 15, 16, 17, 21, 25) sont disposés à l'extérieur du boîtier (2) et reliés par des flexibles d'arrivée avec des conduits d'arrivée et de communication (77, 78, 79, 84, 85, 88, 89, 90, 91, 92, 93 et 94) dans le boîtier (2).

8. Dispositif selon la revendication 1, **caractérisé en ce que** la taille des réservoirs (8, 9, 10, 11, 15, 16, 17) disposés à l'intérieur du boîtier (2) est définie de telle sorte que leur capacité d'enzymes et/ou de réactifs, de solutions tampons et de solutions de calibration soit dimensionnée pour au moins 9660 analyses et **en ce que** la taille des chambres de réaction (3, 4, 5 et 6) est au maximum de 1 cm$^3$.

9. Dispositif selon la revendication 1, **caractérisé en ce qu'**il est prévu dans le boîtier (2) un réservoir (37) pour de l'eau pure et un dispositif de préparation de l'eau (36) relié à celui-ci, auquel de l'eau (100) peut être amenée.

10. Dispositif selon l'une quelconque des revendications 1 à 6, **caractérisé en ce que** les liquides sortant des chambres de réaction (3, 4, 5 et 6) peuvent être amenés à un dispositif de filtration (38) et évacués vers l'extérieur à partir de celui-ci hors du boîtier (2).

11. Dispositif selon la revendication 6, comprenant une source de tension (57) installée à l'extérieur du boîtier (2) et un distributeur de tension (56) disposé dans le boîtier (2) et relié à la source de tension (57), **caractérisé en ce qu'**au moins un microprocesseur (35) est prévu pour la commande des pompes (59, 61, 62, 63, 64, 65, 66, 67, 68, 69, 70, 71) et pour l'enregistrement et la transmission de signaux de mesure des dispositifs de mesure (31, 32 et 33) à un transmetteur (110).

# Fig.1

# Fig.2

# Fig.3

# Fig.4